# EUROPEAN PATENT SPECIFICATION

(11) **EP 1 325 901 B1**
(45) Date of publication and mention of the grant of the patent: **30.11.2005**
(21) Application number: 02000132.7
(22) Date of filing: 04.01.2002
(51) Int. Cl.: C07C 51/31, C07C 55/14

(54) **An improved process for the preparation of adipic acid**
Ein verbessertes Verfahren zur Herstellung von Adipinsäure
Procédé amélioré de préparation d'acide adipique

(43) Date of publication of application: 09.07.2003
(73) Proprietor: COUNCIL OF SCIENTIFIC AND INDUSTRIAL RESEARCH, New Delhi 110 001 (IN)
(72) Inventor: Srinivas, Darbha, Pune - 411, 008 Maharashtra (IN); Chavan, Suhas Arunkumar, 008 Maharashtra (IN); Ratnasamy, Paul,, 008 Maharashtra (IN)
(74) Representative: Maiwald Patentanwalts GmbH

(56) References cited:
- CHAVAN, S.A., ET. AL: "A novel, zeolite-encapsulated m3-oxo Co/Mn cluster catalyst for oxidation of para-xylene to terephthalic acid" CHEMICAL COMMUNICATION, 2001, pages 1124-1125, XP002201884
- CHAVAN, S.A. ET AL: "Formation and role of cobalt and manganese cluster complexes in the oxidation of p-xylene" JOURNAL OF MOLECULAR CATALYSIS A: CHEMICAL, vol. 161, 2000, pages 49-64, XP002201885
- SUMMER C E ET AL: "ISOLATION OF OXO-CENTERED COBALT(III) CLUSTERS AND THEIR ROLE IN THE COBALT BROMIDE CATALYZED AUTOXIDATION OF AROMATIC HYDROCARBONS" JOURNAL OF THE AMERICAN CHEMICAL SOCIETY, AMERICAN CHEMICAL SOCIETY, WASHINGTON, DC, US, vol. 107, 1985, pages 6124-6126, XP001026557 ISSN: 0002-7863
- DATABASE WPI Section Ch, Week 198933 Derwent Publications Ltd., London, GB; Class A41, AN 1989-236342 XP002201886 & JP 01 135741 A (KOREA RES INST CHEM TECH), 29 May 1989 (1989-05-29)

## Description

### Field of the invention

The present invention relates to an improved process for the preparation of adipic acid. More particularly, the present invention relates to an environmental-friendly, clean process for the preparation of adipic acid through oxidation of cyclohexanol, cyclohexanone or a mixture thereof with oxygen or oxygen-containing gas, in the presence of an oxidation initiator, a polar solvent and an organometallic µ₃-oxo-bridged Co/Mn cluster complex catalyst.

### Background of the invention

Adipic acid is a large volume commodity chemical and is used in the manufacture of synthetic fibers (e.g, Nylon-6,6), polyurethane resins, plasticisers, food additives, lubricants, electronics, soil conditioners, glass protection agents and leather tanning agents. More than two million tons of adipic acid is produced worldwide per year, mostly, by a two-step oxidation process (Du Pont U.S. Pat. Nos. 2,233,494 (1940); 2,439,513 (1948); 2,825,742 (1958); 3,035,092 (1962); 3,390,174; BASF British Pat. Nos. 852,523 (1958); 918,900 (1963); British Pat. 1,304,855) wherein cyclohexane, in the first-step, is oxidized with oxygen, at 423 - 433 K, using a cobalt catalyst to form a cyclohexanone and cyclohexanol mixture (KA oil). In the second step the mixture is further oxidized to adipic acid with nitric acid, in the presence of V and Cu-containing catalysts. Conversion levels of cyclohexane, in the first step, are to be maintained below 10% to achieve acceptable selectivity of cyclohexanol-cyclohexanone. The second-step of the reaction (nitric acid oxidation) produces significant toxic nitrogen oxide effluents, and hence, is not environmental-friendly. The oxidation of cyclohexane is one of the least efficient of all the major industrial processes. Methods of improving the selectivity have been the focus of research in this area over the decades. Asahi Chemical Industry (Japan Pat. No. 45-16,444 (1970)) and Gulf (U. S. Pat. No. 4,263,453 (1979)) have developed a direct single-step oxidation process of cyclohexane to adipic acid. In this process cyclohexane is oxidized with air or oxygen, in acetic acid medium and cobalt salt as catalyst, at reaction temperature of 343 -373 K and residence time of 2 - 6 h. Cyclohexane conversion of the order of 50 - 94% and adipic acid selectivity of 70 - 80% have been reported in the single step process. Alternative single-step synthetic routes have been reported by Talsi et al (J. Mol. Catal. 81, 215 (1993)) and Maschmeyer et al (Angew. Chem. Int. Ed. Engl. 36, 1639 (1997)). However, the results on single-step oxidation to adipic acid have been less than satisfactory.

Kulsrestha et al in U. S. Pat. No. 5,547,905 (1996) have reported the preparation of adipic acid using Co and Fe containing homogeneous catalysts. U.S. Pat. No. 6,147,256 (2000) reports oxidation of cyclohexane in liquid phase using Co and Cr containing salts. Sato et al (Science 281, 1646 (1998)) have reported a process for the synthesis of adipic acid by oxidation of cyclohexene with H₂O₂ and solid NaWO₄ catalyst.

Kamath and Chandalia (J. Appl. Chem. Biotechnol. 23, 469-478 (1973)) have reported the preparation of adipic acid by the oxidation of cyclohexanone with air (atmospheric pressure), in the presence of acetic acid and cobalt acetate or manganese acetate salt, at 323-373 K. But the cyclohexanone conversion and adipic acid selectivities were very low. S. H. A. Zaidi (Appl. Catal. 42, 247 (1988)), E. T. Crisp and G. H. Whittfield (Great Britain Patent 2,818,807 (1978)) and T. N. Antonova, G. N. Koshcl, and M. I. Farberov (Uch. Zap. Yaroslav, Tekhnol. Inst. 27, 100 (1971), CA 78(11): 71339p) have also reported the oxidation of cyclohexanone to adipic acid, but with lower conversions and adipic acid yields.

Raja and Ratnasamy in U. S. Pat. No. 5,767,320 (1998) have reported dioxygen oxidation of cyclohexane using pure and zeolite-Y-encapsulated substitute phthalocyanine catalysts; cyclohexanone and cyclohexanol were the selective products of the oxidation reaction. Thomas et al have recently reported the preparation of adipic acid by aerial oxidation of cyclohexane or n-hexane using metal containing aluminopbosphate molecular sieve catalysts (Nature 398, 227-230 (1999); Angew. Chem. Int. Ed. 39(13), 2310-2313 (2000); Angew. Chem. Int. Ed. 39(13), 2313 (2000)). U. S. Pat. Nos. 2,223,493; 2,589,648; 3,390,174; 3,649,689; 3,987,100; 4,263,453; 4,158,739; 4,902,827; 5,321,157 (1994); 5,981,420 (1999), 6,160,183 (2000), 6,258,981(2001) and EP-A-0,694,333 also describe other methods for the preparation of adipic acid. Comprehensive reports on the state-of the-art of adipic acid preparation are available in the reviews by K. Tanaka in CHEMTECH 555-559 (1974) and Hydrocarbon Process 53(11), 114-120 (1974), Castellan et al in Catal. Today 9(3), 237-322 (1991), Schuchardt et al in SYNLETT 713-718 (1993) and Appl. Catal. A. General 211, 1-17 (2001); Partenheimer in Catal. Today 23, 69-158 (1995); Suresh et al in Ind. Eng. Chem. Res. 39, 3958-97 (2000). However, in these reports adipic acid yields are lower than the commercial process and limit their applicability.

The commercial adipic acid manufacturing process has the following disadvantages: (1) the commercial process is not an environmentally benign or "green" approach. (2) nitrous oxide is an inevitable by-product, which has been implicated in global warming and ozone depletion, (3) substantial amount of nitric acid is consumed in the process, (4) decarboxylation to lower mono- and dicarboxylic acids is inevitable, and (4) 0.25 kg of by-products is produced per kg of the product.

The present invention is an environmental-friendly green process. It does not use nitric acid, but utilizes cleaner oxidants like air or oxygen-containing gas. The method of the present invention utilizes an organometallic µ₃-oxo-bridged Co/Mn cluster complex or a solid catalyst containing the organometallic µ₃-oxo-bridged Co/Mn cluster complex as catalyst in a polar solvent medium like acetic acid-water in the presence of an oxidation initiator. Examples of such solid catalysts include micro and mesoporous materials like, aluminosilicate zeolites, aluminophosphates, carbon molecular sieves, silica and the like, containing an organometallic cluster complex wherein the chemical composition of each molecule of the organometallic cluster complex includes cobalt/manganese.

It is a surprising discovery of the present invention that when an organometallic µ₃-oxo-bridged Co/Mn cluster complex or the solid catalyst containing µ₃-oxo-bridged cluster complex was used as catalyst the activity and adipic acid selectivity were significantly higher. In the experiments with the solid catalysts containing the organometallic cluster complex, the solid catalyst can be easily separated from the reaction mixture by filtration.

Moreover the reaction conditions like temperature and pressure were moderate and the process was atom-efficient. The synergistic effect of a cobalt and manganese combination, and facile redox behavior in cluster complexes are perhaps responsible for high yields of adipic acid in the present invention.

Chavan et al in J. Mol. Catal. A. Chemical 161, 49-64 (2000) and Chem. Commun. 1124-1125 (2001) teach that solid, encapsulated oxo-bridgcd metal cluster complexes are efficient catalysts in the aerial oxidation of *para*-xylene to terephthalic acid, an yet another large volume commodity chemical used in polyester industry. These novel solid catalysts while retaining all the advantages of the homogeneous catalysts, like high yield of adipic acid, are easily separable from the reaction products by a simple filtration process. This not only avoids the tedious process of catalyst recovery characteristic of the prior art processes, but also eliminates the presence of toxic metal ions and nitrous oxide in the effluents from the process. Processes utilizing these novel solid catalysts are, hence, environmentally more beneficial. Representatives of the organometallic cluster complexes of cobalt and manganese of the present invention are Co₃(O)(CH₃)COO)₆(py)₃, Mn₃(O)(CH₃COO)₆(py)₃, CoMn₂(O)(CH₃COO)₆(py)₃, Co₂Mn(O)(CH₃COO)₆(py)₃, CoMn₂(O)(CH₃COO)_{y}(py)_{z,} and Co₂Mn(O)(CH₃COO)_{y}(py)_{z,} where y + z = 9 and py = pyridine. It is also found that the organic ligands in the above mentioned organometallic cluster complex, namely the acetate and pyridine ligands, can be replaced by other suitable organic moieties. The critical active site ensemble responsible for the high yields of adipic acids in the oxidation reaction was the heterometallic cluster complex containing both cobalt and manganese. While the exact origin of this enhancement effect is not known in detail, it may be speculated that the multimetallic clusters of transition metal ions are better able to activate oxygen, than the monometallic and monomeric ions. The common prevalence of such heteronuclear, multimetallic clusters in the oxygen activating enzymatic oxygenase catalyst systems supports such a suggestion.

### Objects of the invention

The major object of the present invention is to provide an improved process for the preparation of adipic acid which an environmental-friendly and atom-efficient.

Another objective of the present invention is provide a process for the production of adipic acid wherein nitric acid is not used as an oxidant; cleaner oxidants like oxygen or oxygen-containing gas and a oxo-bridged Co/Mn cluster complex either in its neat form or contained in a solid matrix are used as catalyst.

Yet another objective of the present invention is to prepare adipic acid from cyclohexanol, cyclohexanone or a mixture thereof.

### Summary of the Invention

Accordingly, the present invention provides an improved process for the preparation of adipic acid which comprises oxidizing a cyclic compound selected from the group consisting of cyclohexanol, cyclohexanone and a mixture thereof in a polar solvent with oxygen in the presence of an µ₃-oxo-bridged Co/Mn cluster complex catalyst and an oxidation initiator, at a pressure of at least 997 kPa (130 psig), at a temperarure ranging between 353 and 403 K, for a period ranging from 0.5 to 8.0 hrs, bringing the temparature of reaction mixture to an ambient temperature and recovering the adipic acid from the reaction mixture by conventional methods.

In an embodiment of the present invention the cluster complex has a general formula

[CoₓMn_{y}(O)(OOCR)ₙ]

wherein x and y can take values of 0, 1, 2 or 3 and x + y = 3, R is selected from the group consisting of alkyl, substituted alkyl group containing 1 or more carbon atoms, aryl and substituted aryl group and n is in the range of 4 to 6.

In yet another embodiment the µ₃-oxo cluster complex used is contained in a solid matrix selected from the group consisting of aluminosilicate zeolite, aluminophosphates, carbon molecular sieves and silica..

In yet another embodiment the polar solvent used is a mixture of alkyl or aryl carboxylic acid and water.

In yet another embodiment the oxygen used is selected from the group consisting of pure oxygen, air and a mixture of inert gases and oxygen.

In still another embodiment wherein the oxidation initiator used is selected from the group consisting of methyl ethyl ketone (MEK), acetaldehyde, hydrogen peroxide, alkyl hydroperoxide and halide ion preferably bromide ion.

This invention is illustrated by the following examples, which are illustrative only, and should not be construed to limit the scope of the present invention.

### Preparation of organometallic µ₃-oxo-bridged cluster complexes

The said cluster catalyst has been prepared by a procedure as described and claimed in our co-pending US patent application no. 09/894997.

### EXAMPLE 1

This example illustrates the preparation of cobalt-manganese cluster catalyst CoMn₂(O)(CH₃COO)₆(pyridine)₃ (hereafter designated as CoMn₂(O)). In a typical synthesis, 2.7 g of manganic acetate was taken in a solution containing 25 ml of absolute alcohol and 4.2 ml of glacial acetic acid. The mixture was stirred for 10 min till all the manganic acetate dissolved. To this 2.5 g of cobalt acetate tetrahydrate dissolved in 4 g of hot pyridine was added while stirring continuously. The solution was allowed to stand at 298 K from which shiny black crystals of CoMn₂(O) were obtained.

### EXAMPLE 2

This example illustrates the preparation of manganese cluster complex catalyst Mn₃(O)(CH₃COO)₆(Pyridine)₃ (hereafter designated as Mn₃(O)). Manganese acetate tetrahydrate (2.5 g) was dissolved in a solvent mixture comprising of ethanol (20 ml), glacial acetic acid (12 ml) and pyridine (3 ml). The resulting solution was stirred while adding N-n-Bu₄MnO₄ (1.14 g) dissolved in 10 ml ethanol, in small proportions, over a period of 45 min. The resultant brown solution was stirred for 30 min. Then, 0.695 g of NaClO₄ was added and the stirring was continued for further 15 min. A brown crystalline product of Mn₃(O) was obtained overnight on slow evaporation at 295 K. This was filtered, washed with ethanol and dried in vacuum.

### EXAMPLE 3

This example illustrates the preparation of organometallic cobalt cluster catalyst Co₃(O)(CH₃COO)₆(pyridine)₃ (hereafter designated as Co₃(O)). Cobalt acetate tetrahydrate (1.25 g) was taken in a solution comprising of 12.5 ml of glacial acetic acid and 0.4 ml of pyridine and warmed at 323 K while stirring until all the solid was dissolved. The purple colored solution was then cooled to 298 K and a freshly prepared peracetic acid (obtained from the addition of 0.4 g of glacial acetic acid and 0.7 g of 30% H₂O₂) was added drop-wise over a period of 30 min while stirring. During the addition of peracetic acid the color of the solution changed to dark brown. Then, 3 ml of water was added and refluxed for 1 h at 353 K. A solution of NaClO₄ (0.4 g) dissolved in 20 ml distilled water was added to the cooled reaction mixture. Good quality micro-crystals of catalyst system Co₃(O) were obtained from the solution kept at 278 K.

### EXAMPLE 4

This example illustrates the preparation of the solid catalyst containing the organometallic oxo-bridged Co/Mn cluster complex designated as CoMn₂(O)-Y. Mixed metal Co-Mn(II) exchanged zeolite-HY was prepared by ion-exchange method, in which zeolite HY (7 g) was interacted with 4.3 g of manganese acetate tetrahydrate and 1.43 g of cobalt acetate tetrahydrate dissolved in 100 ml distilled water at 333 K with constant stirring. The solid product was then washed thoroughly with water (500 ml) and dried at 373 K. CoMn-Y thus prepared was taken in 15 ml glacial acetic acid and to this was added pyridine (3 ml), NaBr (0.5 g) and aq. H₂O₂ (50%, 10 ml) and distilled water (5 ml). The reaction mixture was stirred while passing air, for 2 h at 298 K. The purple solid zeolite (CoMn₂(O) cluster complex encapsulated in zeolite-Y; CoMn₂(O)-Y) was then filtered, washed with acetic acid and dried at 298 K under vacuum.

### EXAMPLE 5

This example illustrates the preparation of solid catalyst containing organometallic oxo-bridged Mn cluster catalyst designated as Mn₃(O)-Y. Manganese exchanged zeolite-HY (Mn-Y) was prepared by the ion-exchange method, in which zeolite HY (7 g) was interacted with 4.3 g of manganese acetate tetrahydrate dissolved in 100 ml distilled water at 333 K with constant stirring for 4 - 5 h. The solid product was then filtered, washed thoroughly with water (500 ml) and dried at 373 K. Mn-Y thus prepared was taken in 15 ml glacial acetic acid and to this was added pyridine (3 ml), NaBr (0.5 g) and aq. H₂O₂ (50%, 10 ml) and distilled water (5 ml). The reaction mixture was stirred while passing air, for 2 h at 298 K. The pale brown solid zeolite (Mn₃(O) cluster complex encapsulated in zeolite-Y; Mn₃(O)-Y) was then filtered, washed with acetic acid and dried at 298 K under vacuum.

### EXAMPLE 6

This example illustrates the preparation of the solid catalyst containing organometallic oxo-bridged Co cluster catalyst designated as Co₃(O)-Y. Cobalt exchanged zcolite-HY (Co-Y) was prepared by the ion-exchange method, in which zeolite HY (7 g) was interacted with 4.3 g of cobalt acetate tetrahydrate dissolved in 100 ml distilled water at 333 K with constant stirring for 4 - 5 h. The solid product was then filtered, washed thoroughly with water (500 ml) and dried at 373 K. Co-Y thus prepared was taken in 15 ml glacial acetic acid. To this was added pyridine (3 ml), NaBr (0.5 g) and aq. H₂O₂ (50%, 10 ml) and distilled water (5 ml). The reaction mixture was stirred while passing air, for 2 h at 298 K. The pink solid zeolite (Co₃(O) cluster complex encapsulated in zeolite-Y; Co,(O)-Y) was then filtered, washed copiously with acetic acid and dried at 298 K under vacuum.

### Preparation of adipic acid

### EXAMPLE 7

This example illustrates the procedure for the preparation of adipic acid from cyclohexanone by using cluster catalyst Co₃(O), Mn₃(O) or CoMn₂(O) at 363 K and 3.89 MPa (550 psig) air pressure. The experiments were conducted in a closed titanium-lined pressure reactor (Parr 4843). In a typical oxidation experiment, 4.21 ml of cyclohexanone was taken in 38 ml of glacial acetic acid and 1.9 ml of distilled water. To it 0.114 ml of methylethylketone (MEK) was added. Then 0.354 g of the cluster catalyst (CoMn₂(O), Mn₃(O) or Co₃(O); prepared as reported in Examples 1 -3) was added to the reaction mixture which was then heated to 363 K. This was followed by pressurizing the reactor with air to 3.89 MPa (550 psig) The reaction was conducted for 4 h. Then, the temperature of the reactor was quenched to 293 K using ice. Conversion of cyclohexanone and liquid products distribution was checked by gas chromatographic analysis (Shimadzu GC 14 B, SE-30 S.S. packed column). Later, 2 ml of the reaction mixture was taken and esterified with 5 ml of BF₃ in methanol by refluxing for 8 h. Subsequent treatments of separation yielded the esterified products. These were then dissolved in anhydrous dichloromethane (3 ml) and analyzed by GC (Shimadzu GC 14 B, capillary column).

### EXAMPLE 8

This example illustrates the procedure for the preparation of adipic acid from cyclohexanone using CoMn₂(O) cluster catalyst at 353 K. The experiments were conducted in a closed titanium-lined pressure reactor (Parr 4843). In a typical oxidation experiment, 4.21 ml of cyclohexanone was taken in 38 ml of glacial acetic acid and 1.9 ml of distilled water. To it 0.114 ml of methylethylketone (MEK) was added. Then 0.354 g of CoMn₂(O) prepared as reported in Example 1 was added to the reaction mixture which was then heated to 353 K. This was followed by pressurizing the reactor with air to 3,89 MPa (550 psig) The reaction was conducted for 4 b. Then, the temperature of the reactor was quenched to 293 K using ice. Conversion of cyclohexanone and liquid products distribution was checked by gas chromatographic analysis (Shimadzu GC 14 B, SE-30 S.S. packed column). Later, 2 ml of the reaction mixture was taken and esterified with 5 ml of BF₃ in methanol by refluxing for 8 h. Subsequent treatments of separation yielded the esterified products. These were then dissolved in anhydrous dichloromethane (3 ml) and analyzed by GC (Shimadzu GC 14 B, capillary column).

### EXAMPLE 9

This example illustrates the procedure for the preparation of adipic acid from cyclohexanone using CoMn₂(O) cluster catalyst at 373 K. The experiments were conducted in a closed titanium-lined pressure reactor (Parr 4843). In a typical oxidation experiment, 4.21 ml of cyclohexanone was taken in 38 ml of glacial acetic acid and 1.9 ml of distilled water. To it 0.114 ml of methylethylketone (MEK) was added. Then 0.354 g of CoMn₂(O) prepared as reported in Example 1 was added to the reaction mixture which was then heated to 373 K. This was followed by pressurizing the reactor with air to 3.89 MPa (550 psig). The reaction was conducted for 0.75 or 4 h. Then, the temperature of the reactor was quenched to 293 K using ice. Conversion of cyclohexanone and liquid products distribution was checked by gas chromatographic analysis (Shimadzu GC 14 B, SE-30 S.S. packed column). Later, 2 ml of the reaction mixture was taken and esterified with 5 ml of BF₃ in methanol by refluxing for 8 h. Subsequent treatments of separation yielded the esterified products. These were then dissolved in anhydrous dichloromethane (3 ml) and analyzed by GC (Shimadzu GC 14 B, capillary column).

### EXAMPLE 10

This example illustrates the procedure for the preparation of adipic acid from cyclohexanone at 383 K using CoMn₂(O) cluster catalyst. The experiments were conducted in a closed titanium-lined pressure reactor (Parr 4843). In a typical oxidation experiment, 4.21 ml of cyclohexanone was taken in 38 ml of glacial acetic acid and 1.9 ml of distilled water. To it 0.114 ml of methylethylketone (MEK) was added. Then 0.354 g of CoMn₂(O) prepared as reported in Examples 1 was added to the reaction mixture which was then heated to 383 K. This was followed by pressurizing the reactor with air to 3.89 MPa (550 psig). The reaction was conducted for 4 h. Then, the temperature of the reactor was quenched to 293 K using ice. Conversion of cyclohexanone and liquid products distribution was checked by gas chromatographic analysis (Shimadzu GC 14 B, SE-30 S.S. packed column). Later, 2 ml of the reaction mixture was taken and esterified with 5 ml of BF₃ in methanol by refluxing for 8 h. Subsequent treatments of separation yielded the esterified products. These were then dissolved in anhydrous dichloromethane (3 ml) and analyzed by GC (Shimadzu GC·14 B. capillary column).

The prior-art catalyst, a physical mixture of Co(CH₃COO)₂.4H₂O (0.3106 g) and Mn(CH₃COO)₂.4H₂O) (0.0791 g) was used for comparison with MEK or bromide ion as oxidation initiator.

### EXAMPLE 11

This example illustrates the procedure for the preparation of adipic acid from cyclohexanone using solid µ₃-oxo-bridged organometallic Co/Mn cluster catalyst CoMn₂(O)-Y. The experiments were conducted in a closed titanium-lined pressure reactor (Parr 4843). In a typical oxidation experiment 4.21 ml of cyclohexanone was taken in 38 ml of glacial acetic acid and 1.9 ml of distilled water. To it, 0.114 ml of methylethylketone (MEK) was added. Then 74.9, 149.8, 299.5 or 599.0 mg of CoMn₂(O)-Y catalyst prepared as reported in Example 4 was added. The reaction mixture then pressured with air to 4.93 MPa (700 psig) and then heated to 373 K. The reaction was conducted for 4 h. At the end of the reaction, the temperature of the reactor was quenched to 293 K using ice. The solid catalyst was separated from the reaction mixture by filtration and the conversion of cyclohexanone and liquid products, if any, were checked by gas chromatographic analysis (Shimadzu GC 14 B SE-30 S.S. packed column). Later, 2 ml of the reaction mixture was taken and esterified with 5 ml of BF₃ in methanol by refluxing for 8 h. Subsequent treatments separation yielded the esterified products. These were then dissolved in anhydrous dichloromethane (3 ml) and analyzed by GC (Shimadzu GC 14 B, capillary column). The solid product containing adipic acid was isolated from the solution by distilling out the acetic acid.

### EXAMPLE 12

This example illustrates the procedure for the preparation of adipic acid from cyclohexanone using the solid oxo-bridgcd organometallic Co/Mn cluster catalyst CoMn₂(O)-Y at 3.55 or 6.31 MPa (500 or 900 psig) air pressure. The experiments were conducted in a closed titanium-lined pressure reactor (Parr 4843). In a typical oxidation experiment 4.21 ml of cyclohexanone was taken in 38 ml of glacial acetic acid and 1.9 ml of distilled water. To it, 0.114 ml of methylethylketone (MEK) was added. Then 74.9, 149.8, 299.5 or 599.0 mg of CoMn₂(O)-Y catalyst prepared as reported in Example 4 was added. The reaction mixture then pressured with air to 3.55 or 6.31 MPa (500 or 900 psig) and then heated to 373 K. The reaction was conducted for 4 h. At the end of the reaction, the temperature of the reactor was quenched to 293 K using ice. The solid catalyst was separated from the reaction mixture by filtration and the conversion of cyclohexanone and liquid products, if any, were checked by gas chromatographic analysis (Shimadzu GC 14 B SE-30 S.S. packed column). Later, 2 ml of the reaction mixture was taken and esterified with 5 ml of BF₃ in methanol by refluxing for 8 h. Subsequent treatments separation yielded the esterified products. These were then dissolved in anhydrous dichloromethane (3 ml) and analyzed by GC (Shimadzu GC 14 B, capillary column). The solid product containing adipic acid was isolated from the solution by distilling out the acetic acid.

### EXAMPLE 13

This example illustrates the procedure for the preparation of adipic acid from cyclohexanone using the solid organometallic Mn cluster catalyst Mn₃(O)-Y. The experiments were conducted in a closed titanium-lined pressure reactor (Parr 4843). In a typical oxidation experiment 4.21 ml of cyclohexanone was taken in 38 ml of glacial acetic acid and 1.9 ml of distilled water. To it, 0.114 ml of methylethylketone (MEK) was added. Then 299.5 mg of Mn₃(O)-Y catalyst prepared as reported in Example 5 was added. The reaction mixture then pressured with air to 4.93 MPa (700 psig) and then heated to 373 K. The reaction was conducted for 4 h. At the end of the reaction, the temperature of the reactor was quenched to 293 K using ice. The solid catalyst was separated from the reaction mixture by filtration and the conversion of cyclohexanone and liquid products, if any, were checked by gas chromatographic analysis (Shimadzu GC 14 B SE-30 S.S. packed column). Later, 2 ml of the reaction mixture was taken and esterified with 5 ml of BF₃ in methanol by refluxing for 8 h. Subsequent treatments separation yielded the esterified products. These were then dissolved in anhydrous dichloromethane (3 ml) and analyzed by GC (Shimadzu GC 14 B, capillary column). The solid product containing adipic acid was isolated from the solution by distilling out the acetic acid.

### EXAMPLE 14

This example illustrates the procedure for the preparation of adipic acid from cyclohexanone using the solid organometallic Co cluster catalyst Co₃(O)-Y. The experiments were conducted in a closed titanium-lined pressure reactor (Parr 4843). In a typical oxidation experiment 4.21 ml of cyclohexanone was taken in 38 ml of glacial acetic acid and 1.9 ml of distilled water. To it, 0.114 ml of methylethylketone (MEK) was added. Then 299.5 mg of Co₃(O)-Y catalyst prepared as reported in Example 6 was added. The reaction mixture then pressured with air to 4.93MPa (700 psig) and then heated to 373 K. The reaction was conducted for 4 h. At the end of the reaction, the temperature of the reactor was quenched to 293 K using ice. The solid catalyst was separated from the reaction mixture by filtration and the conversion of cyclohexanone and liquid products, if any, were checked by gas chromatographic analysis (Shimadzu GC 14 B SE-30 S.S. packed column). Later, 2 ml of the reaction mixture was taken and esterified with 5 ml of BF₃ in methanol by refluxing for 8 h. Subsequent treatments separation yielded the esterified products. These were then dissolved in anhydrous dichloromethane (3 ml) and analyzed by GC (Shimadzu GC 14 B, capillary column). The solid product containing adipic acid was isolated from the solution by distilling out the acetic acid.

### EXAMPLE 15

This example illustrates the procedure for the preparation of adipic acid from cyclohexanol using solid µ₃-oxo-bridged organometallic Co/Mn cluster catalyst CoMn₂(O)-Y. The experiments were conducted in a closed titanium-lined pressure reactor (Parr 4843). In a typical oxidation experiment 4.3 ml of cyclohexanol was taken in 38 ml of glacial acetic acid and 1.9 ml of distilled water. To it, 0.114 ml of methylethylketone (MEK) was added. Then 74.9 mg of CoMn₂(O)-Y catalyst prepared as reported in Example 4 was added. The reaction mixture was then pressured with air to 4.93 MPa (700 psig) and heated to 373 K. The reaction was conducted for 4 h. At the end of the reaction, the temperature of the reactor was quenched to 293 K using ice. The solid catalyst was separated from the reaction mixture by filtration and the conversion of cyclohexanol and liquid products were checked by gas chromatographic analysis (Shimadzu GC 14 B SE-30 S.S. packed column). Later, 2 ml of the reaction mixture was taken out and esterified with 5 ml of BF₃ in methanol by refluxing for 8 h. Subsequent treatments yielded the esterified products. These were then dissolved in anhydrous dichloromethane (3 ml) and analyzed by GC (Shimadzu GC 14 B, capillary column). The solid product containing adipic acid was isolated from the solution by distilling out the acetic acid.

### EXAMPLE 16

This example illustrates the procedure for the preparation of adipic acid from a mixture of cyclohexanone and cyclohexanol using solid µ₃-oxo-bridged organometallic Co/Mn cluster catalyst CoMn₂(O)-Y. The experiments were conducted in a closed titanium-lined pressure reactor (Parr 4843). In a typical oxidation experiment 2. 1 ml of cyclohexanone and 2.1 ml of cyclohexanol were taken in 38 ml of glacial acetic acid and 1.9 ml of distilled water. To it, 0.114 ml of methylethylketone (MEK) was added. Then 74.9 mg of CoMn₂(O)-Y catalyst prepared as reported in Example 4 was added. The reaction mixture then pressured with air to 4.93MPa (700 psig) and then heated to 373 K. The reaction was conducted for 4 h. At the end of the reaction, the temperature of the reactor was quenched to 293 K using ice. The solid catalyst was separated from the reaction mixture by filtration and the substrate conversion and liquid products, if any, were checked by gas chromatographic analysis (Shimadzu GC 14 B SE-30 S.S. packed column). Later, 2 ml of the reaction mixture was taken and esterified with 5 ml of BF₃ in methanol by refluxing for 8 h. Subsequent treatments yielded the esterified products. These were then dissolved in anhydrous dichloromethane (3 ml) and analyzed by GC (Shimadzu GC 14 B, capillary column). The solid product containing adipic acid was isolated from the solution by distilling out the acetic acid.

The catalytic data for the oxidation of cyclohexanone, cyclohexanol and a mixture thereof to adipic acid using µ₃-oxo-bridged Co/Mn cluster complex catalysts are listed in Tables 1, 2 and 3.

**Table 1.**

| **Oxidation of cyclohexanone with air over organometallic oxo-bridged cluster catalysts** | | | | | | | |
|---|---|---|---|---|---|---|---|
| Catalyst system | Promoter | Temperature (K) | Time (h) | Conv. Wt. % | Productive selectivity (wt. %) | | |
| | | | | | Succinic acid | Glutaric acid | Adipic acid |
| Co₃(O) | MEK | 363 | 4 | 11.9 | 10.6 | 15.1 | 74.3 |
| Mn₃(O) | MEK | 363 | 4 | 93.7 | 3.0 | 13.9 | 83.1 |
| CoMn₂(O) | MEK | 363 | 4 | 92.2 | 6.9 | 14.7 | 78.4 |
| CoMn₂(O) | MEK | 353 | 4 | 54.4 | 3.1 | 5.7 | 81.2 |
| CoMn₂(O) | MEK | 373 | 0.75 | 66.1 | 3.9 | 15.3 | 80.8 |
| CoMn₂(O) | MEK | 373 | 4 | 97.6 | 1.9 | 11.5 | 86.6 |
| CoMn₂(O) | MEK | 383 | 4 | 56.6 | 24.5 | 16.7 | 58.8 |
| Co+Mn (3:1) | NaBr | 363 | 2 | 89.2 | 9.4 | 16.6 | 74.0 |
| Co+Mn (3:1) | NaBr | 363 | 4 | 94.9 | 6.5 | 14.9 | 78.6 |
| Co+Mn (3:1) | MEK | 363 | 4 | 87.8 | 3.8 | 12.0 | 84.2 |
| Co+Mn (3:1) | NaBr | 363 | 8 | 91.1 | 10.0 | 11.8 | 78.2 |
| Co+Mn (3:1) | MEK | 363 | 8 | 94.7 | 3.6 | 13.1 | 83.3 |

**Table 2.**

| **Oxidation of cyclohexanone to adipic acid with air over solid catalysts containing µ**_{**3**}**-oxo-bridged Co/Mn cluster complexes** | | | | | | | |
|---|---|---|---|---|---|---|---|
| Catalyst system (amount of catalyst) | Promoter | Pressure (psi) (cold) | Conv. wt. % | Productive selectivity (wt. %) | | | |
| | | | | Succinic acid | Glutaric acid | Valeric acid | Adipic acid |
| Co₃(O)-Y (299.5 mg) | MEK | 700 | 10.2 | 0.9 | 6.6 | | 92.5 |
| Mn₃(O)-Y (299.5 mg) | MEK | 700 | 96.8 | 2.8 | 14.1 | 1.6 | 81.5 |
| CoMn₂(O)-Y (74.9 mg) | MEK | 700 | 98.3 | 1.8 | 10.9 | - | 87.3 |
| CoMn₂(O)-Y (149.8 mg) | MEK | 700 | 98.4 | 3.90 | 15.50 | 1.30 | 79.3 |
| CoMn₂(O)-Y (299.5 mg) | MEK | 500 | 95.2 | 1.37 | 10.83 | - | 87.8 |
| CoMn₂(O)-Y (299.5 mg) | MEK | 700 | 98.4 | 4.52 | 16.52 | 1.97 | 77.0 |
| CoMn₂(O)-Y (299.5 mg) | MEK | 900 | 94.8 | 3.38 | 14.27 | 2.53 | 79.8 |
| CoMn₂(O)-Y (599.0 mg) | MEK | 700 | 92.7 | 4.13 | 16.51 | 0.144 | 79.2 |

**Table 3.**

| **Oxidation of cyclohexanone/cyclohexanol to adipic acid over CoMn**_{**2**}**(O)-Y catalyst** | | | | | | | | |
|---|---|---|---|---|---|---|---|---|
| Run No. | Substrate | Initiator | Conv (wt.%) | Product distribution (wt.%) | | | | |
| | | | | Cyclohexanone | SA | GA | VA | AA |
| 1 | Cyclohexanone + Cyclohexanol (50 :50) | MEK | 89.6 | 0 | 4.3 | 14.1 | 0.2 | 81.4 |
| 2 | Cyclohexanol (100) | MEK | 45.0 | 39.0 | 4.1 | 0 | 0 | 1.9 |

The process described above has the combined unique advantages of high conversion of cyclohexanone accompanied with high selectivity of adipic acid and low concentration of other by-product impurities. The process is environmental-friendly and results in no undesired toxic effluents.

## Claims

1. A process for the preparation of adipic acid which comprises oxidizing a cyclic compound selected from the group consisting of cyclohexanol, cyclohexanone and a mixture thereof in a polar solvent with oxygen in the presence of an µ₃-oxo-bridged Co/Mn cluster complex catalyst and an oxidation initiator, at a pressure of at least 997 KPa (130 psig) at a temperature ranging between 353 and 403 K, for a period ranging from 0.5 to 8.0 hrs, bringing the temparature of reaction mixture to an ambient temperature and recovering the adipic acid from the reaction mixture by conventional methods.

2. A process as claimed in claim 1 wherein the cluster complex has a general formula
[CoₓMn_{y}(O)(OOCR)ₙ]
wherein x and y can take values of 0, 1, 2 or 3 and x + y = 3, R is selected from the group consisting of alkyl, substituted alkyl group containing 1 or more carbon atoms, aryl and substituted aryl group and n is in the range of 4 to 6.

3. A process as claimed in claims 1 and 2, wherein the µ₃-oxo cluster complex used is contained in a solid matrix selected from the group consisting of aluminosilicate zeolite, aluminophosphates, carbon molecular sieves and silica..

4. A process as claimed in claims 1-3, wherein the polar solvent used is a mixture of alkyl or aryl carboxylic acid and water.

5. A process as claimed in claims 1-4, wherein the oxygen used is selected from the group consisting of pure oxygen, air and a mixture of inert gases and oxygen.

6. A process as claimed in claim 1, wherein the oxidation initiator used is selected from the group consisting of methyl ethyl ketone (MEK), acetaldehyde, hydrogen peroxide, alkyl hydroperoxide and halide ion preferably bromide ion.

## Patentansprüche

1. Ein Verfahren zur Herstellung von Adipinsäure, das das Oxidieren einer cyclischen Verbindung, die aus der Gruppe, bestehend aus Cyclohexanol, Cyclohexanon und einer Mischung daraus, ausgewählt ist, in einem polaren Lösemittel mit Sauerstoff in der Gegenwart eines µ₃-oxo-verbrückten Co/Mn-Cluster-Komplex-Katalysators und eines Oxidationsinitiators bei einem Druck von wenigstens 997 KPa (130 psig) und einer Temperatur zwischen 353 und 403 K, für eine Zeitspanne von 0,5 bis 8 Stunden, das Bringen der Temperatur der Reaktionsmischung auf eine Umgebungstemperatur und das Gewinnen der Adipinsäure durch konventionelle Methoden aus dem Reaktionsgemisch, umfasst.

2. Ein Verfahren wie in Anspruch 1 beansprucht, wobei der Cluster-Komplex eine allgemeine Formel
(CoₓMn_{y}(O)(OOCR)ₙ]
aufweist, wobei x und y Werte von 0, 1, 2 oder 3 annehmen können und x + y = 3 ist, R aus der Gruppe bestehend aus Alkyl, substituierter Alkyl-Gruppe enthaltend ein oder mehr Kohlenstoffatome, Aryl und substituierter Aryl-Gruppe ausgewählt ist und n im Bereich von 4 bis 6 ist.

3. Ein Verfahren wie in Ansprüchen 1 und 2 beansprucht, wobei der verwendete µ₃-oxo-Cluster-Komplex in einer festen Matrix enthalten ist, die aus der Gruppe bestehend aus Aluminosilikat-Zeoliten, Aluminophosphaten, Kohlenstoff-Molekularsieben und Silica ausgewählt ist.

4. Ein Verfahren wie in Ansprüchen 1 bis 3 beansprucht, wobei das verwendete polare Lösemittel eine Mischung aus Alkyl - oder Arylcarbonsäure und Wasser ist.

5. Ein Verfahren wie in Ansprüchen 1 bis 4 beansprucht, wobei der verwendete Sauerstoff aus der Gruppe bestehend aus reinem Sauerstoff, Luft und einer Mischung aus inerten Gasen und Sauerstoff ausgewählt ist.

6. Ein Verfahren wie in Anspruch 1 beansprucht, wobei der verwendete Oxidationsinitiator aus der Gruppe bestehend aus Methylethylketon (MEK), Acetaldehyd, Wasserstoffperoxid, Alkylhydroperoxid und Halogen-Ionen, vorzugsweise Bromidionen, ausgewählt ist.

## Revendications

1. Procédé pour la préparation d'un acide adipique qui comporte l'oxydation d'un composé cyclique sélectionné parmi le groupe constitué de cyclohexanol, de cyclohexanone et d'un mélange de ceux-ci dans un solvant polaire avec de l'oxygène en présence d'un catalyseur complexe d'agrégat Co/Mn à pont µ₃-oxo et d'un initiateur d'oxydation, à une pression d'au moins 997 kPa (130 psig) à une température dans la plage comprise entre 353 et 403°K, pendant une période dans la plage de 0,5 à 8,0 h, en amenant la température du mélange de réaction à une température ambiante et en récupérant l'acide adipique à partir du mélange de réaction par des procédés classiques.

2. Procédé selon la revendication 1, dans lequel le complexe d'agrégat a une formule générale suivante
[CoₓMn_{y}(O)(OOCR)ₙ]
où x et y peuvent adopter des valeurs de 0, 1, 2 ou 3 et x + y = 3, R est sélectionné parmi le groupe constitué d'un alkyle, d'un groupe alkyle substitué contenant 1 ou plusieurs atomes de carbone, d'un aryle et d'un groupe aryle substitué et n se trouve dans la plage allant de 4 à 6.

3. Procédé selon la revendication 1 ou 2, dans lequel le complexe d'agrégat µ₃-oxo utilisé est contenu dans une matrice solide sélectionnée parmi le groupe constitué d'une zéolite d'aluminosilicate, d'aluminophosphates, de tamis moléculaires carbonés et de silice.

4. Procédé selon les revendications 1 à 3, dans lequel le solvant polaire utilisé est un mélange d'acide carboxylique d'alkyle ou d'aryle et d'eau.

5. Procédé selon les revendications 1 à 4, dans lequel l'oxygène utilisé est sélectionné parmi le groupe constitué d'oxygène pur, d'air et d'un mélange de gaz inertes et d'oxygène.

6. Procédé selon la revendication 1, dans lequel l'initiateur d'oxydation utilisé est sélectionné parmi le groupe constitué de méthyléthylcétone (MEK), d'acétaldéhyde, de peroxyde d'hydrogène, d'hydroperoxyde d'alkyle et d'un ion halogénure, de préférence un ion bromure.
